# EUROPEAN PATENT SPECIFICATION

(11) **EP 4 326 280 B1**
(45) Date of publication and mention of the grant of the patent: **12.11.2025**
(21) Application number: 21937783.5
(22) Date of filing: 24.04.2021
(51) Int. Cl.: A61K 31/7048, A61K 36/48, A61K 9/08, A61K 9/20, A61P 7/06

(54) **PHARMACEUTICAL COMPOSITIONS COMPRISING PICROSIDE**
PHARMAZEUTISCHE ZUSAMMENSETZUNGEN MIT PICROSIDE
COMPOSITIONS PHARMACEUTIQUES COMPRENANT DU PICROSIDE

(43) Date of publication of application: 28.02.2024
(73) Proprietor: Goldlake Lifesciences Pvt Ltd, Pune 411026 (IN)
(72) Inventor: JADHAV, Vinod Ramchandra, Dubai, 388-097 (AE); PADMANABHAN, Sriram, Pune 411033 (IN)
(74) Representative: JD&P Patent Attorneys Joanna Dargiewicz & Partners
(86) International application number: PCT/IB2021/053392
(87) International publication number: WO 2022/224025

(56) References cited:
- WO-A1-2020/165664
- CN-A- 101 194 911
- CN-A- 112 237 585
- IN-A- 201721 000 684
- US-A1- 2016 051 612
- SHRINIVAS G. BHOPE ET AL: "A NOVEL APPROACH FOR THE QUALITY ASSESSMENT AND STABILITY TESTING OF AYURVEDIC POLYHERBAL FORMULATIONS BY HPTLC FINGERPRINT METHOD", JOURNAL OF LIQUID CHROMATOGRAPHY AND RELATED TECHNOLOGIES, vol. 34, no. 8, 12 April 2011 (2011-04-12), US, pages 579 - 590, XP055159930, ISSN: 1082-6076, DOI: 10.1080/10826076.2010.534723

## Description

### TECHNICAL FIELD

The present invention relates to a composition comprising a purified compound isolated from *Picrorrhiza kurroa.* Specifically, the present invention relates to a composition comprising purified Picroside isolated from *Picrorrhiza kurroa.* More specifically, the present invention relates to a composition comprising purified Picroside I for preventing or treating immunopathic condition such as neutropenia and the use thereof.

### BACKGROUND

Drug-induced neutropenia, an immunopathic condition, is a potentially serious and life-threatening adverse event that may occur secondary to therapy with a variety of agents. Mostly caused by cytotoxic chemotherapy, neutropenia is marked by a predictable and dose-related decrease in neutrophil count. It has been associated with a variety of chemotherapeutic and non-chemotherapeutic medications including, but not limited to, cyclophosphamide, thionamides, clozapine, dapsone, methimazole, penicillin, rituximab, procainamide, sulfasalazine, thiamazole, carbimazole, amoxicillin, cotrimoxazole, ticlopidine, and valganciclovir. Neutropenia from non chemotherapy drugs is less common than neutropenia secondary to chemotherapy. While the condition can occur at any time during the course of treatment, depending on the primary therapy, it most commonly occurs within the first few weeks after administration of drug. Affected patients typically experience severe neutropenia for several weeks to several months after first exposure to a drug. Drug-induced neutropenia is characterized by decreased production or increased destruction of neutrophils. The clinical consequence of neutropenia is susceptibility to infection proportional to the degree of neutropenia, and can even lead to mortality.

Granulocyte Colony Stimulating Factor (G-CSF) and Granulocyte-macrophage colony-stimulating factor (GM-CSF) are approved by the United States' Food and Drug Administration (FDA) for use treat severe neutropenia with fever. G-CSFs and GM-CSFs have demonstrated proven efficacy in their ability to reduce the incidence, magnitude, and duration of neutropenia following chemotherapy administration; and therefore, are largely and widely accepted treatment options for drug-induced neutropenia. However, neither drugs are not without adverse effects. Some common symptoms observed after administration of G-CSF are dyspnea, chest pain, nausea, hypoxemia, diaphoresis, anaphylaxis, syncope and flushing. In comparison, the adverse effects of GM-CSFs are moderate such as fever, myalgia, malaise, rash and injection site reaction; but severe effects such as pericarditis and thrombosis at high doses are also known to happen. This raises the need for improved therapeutical options, preferably derived from natural resources with enhanced safety and efficacy.

In recent decades, spectrum of disease has shifted to complex chronic diseases requiring aggressive therapies such as chemotherapy. Synthetic drugs generally have more associated adverse effects and toxicities which add on to patients' burden. World Health Organization Drugs Strategy 2002-2005 encouraged acceptance of herbal medicine as complimentary or alternate medicine across the globe. As per WHO data approximately 70% of the world's population has been using herbal medicine as a complementary medicine over the past 2 decades. Herbal medicines are often administered simultaneously with therapeutic drugs for the treatment of major ailments owing to the general perception that they are free of undesirable side effects.

*Picrorhiza kurroa* is a well-known medicinal herb, traditionally popular in Indian ayurvedic system for the treatment of liver and respiratory disorders. Picroside I and Picroside II are two of the most important constituents found in the roots and rhizomes of the plant. Picroside I and Picroside II have been used in herbal formulations individually and in combination. Pharmacological benefits of Picroside I and Picroside II have been widely reported in literature as an antioxidant, antiinflammatory, anti-allergic, hepatoprotective, choleretic, anti-asthmatic and anti-cancerous activity. However, there has not been reported a composition comprising Picroside I, or Picroside II, or a combination thereof for treating neutropenia.

Accordingly, the present inventors report a novel pharmacological benefit of Picroside in the treatment of neutropenia. The disclosed invention is focused on developing a composition comprising active ingredient derived from natural resources for treating neutropenia. According to the present invention, there is therefore provided a composition comprised of Picroside for treating neutropenia. More particularly, there is provided a composition comprising Picroside I for the treatment of neutropenia. Further disclosed is a novel industrialized method for isolating Picroside I and Picroside II. The present invention also discloses a method for making the said novel pharmaceutical composition comprising Picroside. The present invention also provides a method of treating or preventing neutropenia in a subject comprising administering to said subject a therapeutically effective amount of the novel composition containing active ingredient such as Picroside I.

### SUMMARY OF THE INVENTION

In view of the foregoing, for overcoming the disadvantages of the prior art, the object of the invention is to provide a pharmaceutical composition comprising a therapeutically effective amount of Picroside I for the treatment or prevention of neutropenia.

To achieve the above, the present invention includes a composition comprising purified Picroside I and pharmaceutically acceptable excipients for treating or preventing neutropenia in a subject in need thereof. Further, the said composition shall be in oral or injectable form.

According to one embodiment, is provided a neutropenia treating pharmaceutical composition comprising a therapeutically effective amount of purified Picroside I and acceptable pharmaceutical carriers or excipients.

According to one embodiment, the pharmaceutical composition comprises 100 to 400 mg of purified Picroside I and acceptable pharmaceutical carriers or excipients.

According to a preferred embodiment, the amount of purified Picroside I in the said pharmaceutical composition is 150 to 400 mg.

According to one embodiment, the pharmaceutical composition is an oral or injectable composition.

According to a preferred embodiment, the oral pharmaceutical composition is in form of a tablet.

According to one embodiment, the pharmaceutical composition further comprising a filler, a solvent, and a lubricant. According to another embodiment, the filler is F melt Type C, the solvent is ethanol, the lubricant is magnesium stearate. According to a preferred embodiment the amount of filler is about 98 percent by weight, the amount of lubricant is about 1 percent by weight.

According to one important embodiment, is provided a neutropenia treating pharmaceutical composition comprising 390 mg of purified Picroside I, a filler, a solvent, a lubricant; wherein the said composition is ready for oral administration.

According to an alternate embodiment, the injectable pharmaceutical composition is in form of an injection.

According to one embodiment, the pharmaceutical composition further comprising a solvent. According to a preferred embodiment, the solvent is 2.5% PEG400.

According to one important embodiment, is provided a neutropenia treating pharmaceutical composition comprising 195 mg of purified Picroside I, and a solvent; wherein the said composition is sterile and ready for subcutaneous having pH in the range of 4.0 to 7.0.

According to one embodiment, the injectable pharmaceutical composition is stored in a pre -filled sterile syringe or vial or cartridge.

According to one important embodiment, is provided a method of treating neutropenia in a subject comprising administering to a subject in need thereof a therapeutically effective amount of Picroside I comprised in the said composition.

According to one embodiment, the composition is administered to the subject once a day. According to another embodiment, the composition is administered to the subject for up to 5 consecutive days.

### DETAILED DESCRIPTION

To clarify the above and other purposes, features, and advantages of this invention, specific embodiment of this invention is especially listed and described in detail with the examples as follows. The principal and mode of operation of this invention have been described and illustrated in its embodiment. At the outset, a person skilled in the art will appreciate that this invention may be practiced otherwise than is specifically described and illustrated. The invention should not be limited by the above-described embodiment, method, and examples, but by all embodiments and methods within the scope and spirit of the invention. Also, in the following description of the invention, certain terminology may be used for the purpose of reference only, and is not intended to be limiting.

Where a range of values is provided, it is understood that each intervening value, to the tenth of the unit of the lower limit unless the context clearly dictates otherwise, between the upper and lower limits of that range is also specifically disclosed. Each smaller range between any stated value and/or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

The present invention provides a pharmaceutical composition comprising a Picroside compound, derived from *P. kurroa* for the treatment or prevention of immunopathic conditions such as neutropenia. More particularly, the present invention provides a pharmaceutical composition comprising Picroside I for the treatment or prevention of neutropenia. Further, the present invention provides a novel industrialized method for isolating Picroside I and Picroside II from *P. kurroa.* The present invention also provides a method of treating or preventing neutropenia in a subject comprising administering to said subject a therapeutically effective amount of the novel composition comprising active ingredient Picroside I, together with pharmaceutically acceptable carrier or additives thereof.

Accordingly, it is an object of the present invention to provide a pharmaceutical composition comprising Picroside I for treating or preventing neutropenia in a subject suffering from neutropenia by administering to said subject a therapeutically effective amount of the said composition comprising active ingredient Picroside I, together with a pharmaceutically acceptable carrier or additive thereof.

The term "Picroside I" disclosed herein comprises Picroside I isolated from *P. kurroa.* The term *"Picrorhiza kurroa"* or *"P. kurroa"* disclosed herein comprises the cultivated or naturally grown plant and commercially available plant, but not intended to limit thereto herein. Picrorhiza kurroa Royle ex Benth., commonly known as 'Kutki or Kutaki', is a perennial herb from the Scrophulariacae family, present in wild form in the north-western Himalayan regions and Garhwal regions of India. *P. kurroa* is listed as an official drug since 1970 in The Pharmacopoeia of India, 1970. *P. kurroa* is commercially available in India was thus procured. Accordingly, in the present invention, the plant raw material and extract were commercially procured. The roots of *Picrorhiza kurroai* were procured from Noornihal, Anekal, Bangalore; batch number RM/PK/SEP-19-03. Water extract of *Picrorhiza kurroai* were procured from Noornihal, Anekal, Bangalore; batch number RDP/PK/011.

It should be noted that herbs and plants can be processed and can be taken in different ways and forms, and this may include whole injectables, capsules, and tablets that contain a ground or powdered form of a raw herb or its dried extract. Accordingly, a part of the plant may be used as a raw material, or a liquid and/or solid extract thereof Suitable procedures include multiple steps, for example maceration, percolation, and extraction using supercritical fluids, liquefied gases or suitable solvents are used for the preparation of the material. Picroside I and Picroside II are derived from roots and rhizomes of *P. kurroa.* The constituents need to be extracted from the plant material, further purified and isolated in order to make it fit for the designated use in a composition for treating or preventing neutropenia. An aspect of the present invention is a method for extracting, further purifying, and isolating Picroside I and Picroside II from *P. kurroa* to obtain purified Picroside I and Picroside II.

Accordingly, in an embodiment of the present invention, pharmaceutical composition of the present invention comprises of purified Picroside I. And accordingly, there is provided a method for extracting, further purifying, and isolating Picroside I and Picroside II from *P. kurroa* to obtain purified Picroside I and Picroside II. Disclosed herein is the method for extracting Picroside I and Picroside II from P. *kurroa* comprising the steps of: making a coarse powder of *P. kurroa* roots and rhizomes and then using an extracting solvent selected from water, demineralized water, methanol, ethanol in different ratios. etc. or the mixtures thereof at a pre-determined pH; performing at least one more extraction cycle using the said solvent at a pre-determined temperature; collecting and filtering the liquid extract; evaporation of liquid extract under vacuum at pre-determined temperature to obtain a dry powder extract. Disclosed herein is also the method for further purifying Picroside I and Picroside II from *P. kurroa* dry powder extract comprising the steps of: dissolving the dry powder extract in water, loading on using ion exchange column chromatography methods using resins; collection of the first fraction in water; collection of at least two more subsequent fractions in a methanol; washing the column with methanol; and drying the concentrate comprising of Picroside I and Picroside II. The method of isolating Picroside I and Picroside II from P. kurroa comprises the steps of: using silica column chromatography; dissolving the said extracted and purified extract in methanol; collecting at least one fraction comprising Picroside I or Picroside II in methanol and chloroform; using thin-layer chromatography for eluting Picroside I and Picroside II.

Specifically, disclosed herein is the method for extracting Picroside I and Picroside II from *P. kurroa* comprising the steps of: extracting coarse powder of *P. kurroa* roots and rhizomes using demineralized water as the extracting solvent selected from water, wherein the pH ranges from 6.5-7.5; performing at least one more extraction cycle using the said solvent at temperature ranging between 50 -90°C; collecting and filtering the liquid extract; evaporation of liquid extract under vacuum at temperature ranging between 40 -55°C to obtain a dry powder extract characterized by about 28.75 % extract yield. The method for further purifying Picroside I and Picroside II from *P. kurroa* comprises the steps of: using reverse phase chromatography methods including using resins selected from Diaion-HP-20, Amberlite-XAD-4,8,7,16 etc. Preferably, resins that are suitable for preliminary purification of polar compounds like phenolics.etc. are selected. The loaded column is repeatedly washed with water till the colourless eluates are obtained. Further, the column is eluted with 30% methanol in water and fractions collected. This step is repeated till colourless eluates are obtained. Subsequently, the column is washed with 100% methanol and fractions of about 100 ml are collected. The fractions are checked by TLC for markers of interest under UV and selected fractions are dried to obtain the concentrate comprising of about 4 to 6% Picroside I and about 5 to 8% Picroside II.

The dried fraction is resuspended in methanol, adsorbed on silica where 2 volumes of silica is added to the methanol Picroside mixture, and dried by rotavapor. The dried material is then loaded onto a silica column that is packed with chloroform. The column is washed with chloroform, 2% methanol in chloroform followed by 3% methanol in chloroform and finally 4% methanol in chloroform. This method provides pure Picroside I and Picroside II in 4% methanol in chloroform fraction. Fractions containing minimal contaminants and maximum Picroside are chosen and dried and weighed. The final weight is then calculated for determining total yield. The purity is then checked by HPLC. By this process, the Picroside I elutes first followed by Picroside II, characterized by containing about 96.42% and about 89.02% purity respectively.

The present inventors report that the above-described process of the present invention has a shorter time of 3 hours for purification of Picroside I as compared to the process published in the prior art (Hussain A et al., Pharmacognosy Research, 2013; 5:30-36) requiring 6 hours; while still resulting in a marginally higher yield. Additionally, the process of the present invention utilizes a single solvent i.e., water, a safer medium, as compared to the use of two environmental non-friendly solvents such as petroleum ether, ethanol etc. as reported by the prior art (Hussain et al., 2013)

The present inventors report that the said purified Picroside I and Picroside II compounds exhibit the ability to treat and prevent immunopathic conditions namely neutropenia through various *in vivo* tests in mice. To the best of knowledge, this anti-neutropenia activity of Picroside I or Picroside II has not been reported before. It was further observed that Picroside I exhibit marginally more desirable activity for treating or preventing neutropenia as compared to Picroside II. Additionally, mice treated with Picroside I exhibited higher amount of WBC as compared to those treated with Picroside II. Accordingly, Picroside I was selected as the candidate for composition for treating neutropenia owing to its overall effect on neutrophil and WBC amounts. Central to this invention is the object to provide a pharmaceutical composition of Picroside I to treat or prevent neutropenia. Accordingly, in accordance with the other aspect of the present invention, present invention provides a pharmaceutical composition comprising the purified Picroside I prepared by the above-described methods to treat or prevent neutropenia. A pharmaceutical composition of Picroside II is also disclosed herein.

The term "treat" disclosed herein comprises any act to alleviate or favorably changing the symptom associated with certain disease or disorder disclosed herein by way of administrating the said composition; and the term "prevent" disclosed herein comprises any act to inhibit or postpone the occurrence of certain disease or disorder disclosed herein by way of administrating the said composition.

An embodiment of the present invention provides a pharmaceutical composition comprising purified Picroside I prepared by the above-described methods and pharmaceutically acceptable carriers or excipients, for the treatment or prevention of neutropenia. The term "pharmaceutically acceptable carriers or excipients" defined herein comprises pharmaceutical additives, inactive ingredients, dyes, flavors, binders, emollients, fillers, lubricants, preservatives, and many more classifications used to prepare medications and which are well-known in the art or previous literature.

In an embodiment of the present invention, the pharmaceutical composition comprising purified Picroside I may be prepared as an oral dosage form for example, powder, tablet, capsule, soft capsule, aqueous medicine, syrup, elixirs pill, powder, sachet, granules. In a preferred embodiment, the pharmaceutical composition of the present invention in an oral dosage shall be a tablet. In another embodiment of the present invention, the pharmaceutical composition shall further comprise of pharmaceutically acceptable excipients such as fillers, solvent, and lubricant. In a preferred embodiment, the filler shall be F melt Type C, the solvent shall be ethanol, and the lubricant shall be magnesium stearate. It should be noted that the amounts of excipients shall be in proportion to the amount of active ingredient i.e., Picroside I. Accordingly, the amount of filler shall be about 98 % by weight, the amount of lubricant shall be about 1% by weight.

Thus, according to an embodiment of the present invention is provided a neutropenia treating pharmaceutical composition comprising 390 mg of purified Picroside **I, a** filler, a solvent, a lubricant; wherein the said composition is ready for oral administration.

The composition according to the present invention can also be provided as a pharmaceutical composition comprising pharmaceutically acceptable carriers, adjuvants or diluents, e.g., lactose, dextrose, sucrose, sorbitol, mannitol, xylitol, erythritol, maltitol, starches, acacia rubber, alginate, gelatin, calcium phosphate, calcium silicate, cellulose, methyl cellulose, polyvinyl pyrrolidone, water, methylhydroxy benzoate, propylhydroxy benzoate, talc, magnesium stearate and mineral oil. The composition may additionally include anti-agglutinating agents, wetting agents, flavoring agents, emulsifiers, preservatives, etc. The compositions of the invention may be formulated so as to provide quick, sustained or delayed release of the active ingredient after their administration to a patient by employing any of the procedures well known in the art.

In an alternate embodiment, the pharmaceutical composition comprising purified Picroside I may be prepared as an injectable preparation for example, solution, suspension, or emulsion. In a preferred embodiment, the pharmaceutical composition of the present invention shall be an injection. In another embodiment of the present invention, the pharmaceutical composition shall further comprise of pharmaceutically acceptable solvent selected from methanol, 2.5% PEG400, 5% PEG400, 5% DMSO, 5% ethyl lactate, and 1.4% propylene glycol. In a preferred embodiment, the said composition shall comprise of 2.5% PEG400 wherein the pH of the composition shall be in the range of 4.0 to 7.0. It should be noted that the injectable compositions of the present invention can also be dissolved in oils, or other solvents that are commonly used to produce an injection. Suitable examples of the carriers include physiological saline, ethanol, vegetable oils, isopropyl myristate, etc., but are not limited to them. In another embodiment of the present invention, the injectable composition shall be ensured for sterility and ready for use; wherein the composition can be stored in a pre-filled syringe or vial or cartridge or any other suitable mode or medium.

Thus, according to an embodiment of the present invention is provided a neutropenia treating pharmaceutical composition comprising 195 mg of purified Picroside **I,** and a solvent; wherein the said composition is sterile and ready for subcutaneous having pH in the range of 4.0 to 7.0.

Hereinafter, the formulating methods and kinds of excipients will be described, but the present invention is not limited to them. The representative preparation examples were described as follows.

In accordance with another aspect of the present invention, there is also provided a method of treating or preventing neutropenia in subject, wherein the method comprises administering a composition comprising therapeutically effective amount of the purified Picroside I prepared by the above-described methods into the subject suffering from neutropenia.

Accordingly, in an embodiment of the present invention, there is provided a method of treating or preventing neutropenia in a subject, wherein the method comprises administering a composition comprising therapeutically effective amount of the purified Picroside I prepared by the above-described methods into the subject suffering from neutropenia.

In a preferred embodiment is provided a method of treating neutropenia in a subject comprising administering to a subject in need thereof a therapeutically effective amount of Picroside I as an oral composition or as an injectable composition. In a preferred embodiment, the said composition is administered to the subject once a day. In another preferred embodiment, the said composition is administered to the subject for up to 5 consecutive days.

In accordance with another aspect of the present invention, there is also provided a use of a composition comprising purified Picroside I prepared by the above-described methods and pharmaceutically acceptable carriers or excipients, for manufacture of medicines employed for treating or preventing neutropenia.

In accordance with another aspect of the present invention, the pharmaceutical composition of present invention can be administered to a subject via various routes. In accordance with an embodiment of the present invention. The term "subject" defined herein comprises animal subjects such as rat, mouse, domestic animals or human. Several modes of administration are contemplated, for example, administration can be made orally, or by intravenous, intramuscular, subcutaneous, or intracutaneous injection. It will be apparent to those skilled in the art that various modifications and variations can be made in the compositions, use and preparations of the present invention without departing from the spirit or scope of the invention.

In accordance with an aspect of the invention, the desirable dose of Picroside I may vary depending on the condition and the weight of the subject, severity, drug form, route and period of administration, and may be chosen by those skilled in the art. It should be noted that extrapolation of dose from animals to humans using allometric scaling has been done to assess the initial dose in humans taking into consideration the difference in pharmacokinetics and pharmacodynamics among species. For example, a 1 mg and 2 mg dose for administration in mice is extrapolated as 195 mg and 390 mg dose for administration in humans respectively.

Accordingly, an embodiment of the invention provides that in order to obtain desirable effects, it is generally recommended to administer, in humans, at the amount ranging from 100 to 200 mg/day, preferably 150 to 200 mg/day, more preferably 190 to 200 mg/day of the purified Picroside I compound of the present invention. The dose may be administered in single or divided into several times per day. Such administration shall be done for 5 consecutive days.

Since neutropenia is directly linked to neutrophil count, effect of the composition on the condition is reported specifically via neutrophil and also via WBC count. Accordingly, in order to determine the effect of Picroside I on neutropenia, *in vivo* tests were performed on mice. Neutropenia was induced in mice by administering cyclophosphamide to create an animal test model. The neutropenia induced mice were administered with composition comprising Picroside I and observed for changes in body weight, blood analysis to monitor neutrophil counts, and clinical symptoms such as toxicity and mortality. The study model was replicated in neutropenia induced mice by administering composition comprising Picroside II. The present inventors report that both Picroside I and Picroside II significantly reversed cyclophosphamide induced neutropenia in mice. Further, the efficacy and activity of Picroside I for treatment of neutropenia was compared with that of G-CSF which is the FDA approved drug for treating the condition. The present inventors report that the results of Picroside I treatment in subject mice were comparable to that of G-CSF treatment.

As described in the present invention, inventive composition of purified Picroside I derived from P. *kurroez* showed potent anti-neutropenic activity through *in vivo* tests using by swiss albino mice with drug-induced neutropenia, for example, a test on the proliferation and activity of neutrophils caused by neutropenia occurrence; Therefore, it can be used as the therapeutics for treating and preventing neutropenia.

### EXAMPLES

The following examples are provided for illustrative purposes only, and are intended to be purely exemplary of the disclosure and are not intended to limit the scope of the claims provided herein.

### Example 1. Preparation of the crude extract of P. kurroa

400 gm coarse powder of *Picrorhiza kurroa* rhizomes was extracted with demineralized water at pH 6.5-7.5. About 2000 ml water was added for each cycle. A total three cycles were performed at 80°C. Liquid extract was collected and filtered through a PP-filter cloth to remove the debris. The subsequent filtered liquid extract was evaporated under vacuum at 50- 55°C by using rotary evaporator to obtain a dry powder of extract. Component analysis was performed using HPLC. HPLC result confirmed that the crude extract of *Picrorhiza kurroa* contained about 4 to 6 % Picroside I and about 5 to 8% Picroside II.

### Example 2. Preparation of the purified extract Picroside I and Picroside II of P. kurroa

The crude extract of *P. kurroa* was prepared by the extraction method according to Example 1. 100 ml of HP20 resin was loaded on column and equilibrate with water. 13 gm of water extract of *P. kurroa* was completely dissolved in 100 ml water and adsorbed on resin. First fraction was collected with 300 ml water. Second fraction was collected with 30% Methanol in 300 ml water. Third fraction was collected with 60% Methanol in 400 ml water. The column was washed with Methanol. It was observed that the third fraction and methanol washing contained both Picroside-I and Picroside II. Both fractions were mixed together and dried to obtain a concentrate.

Component analysis was performed using HPLC. HPLC result confirmed that the purified extract of *P. kurroa* contained about 14.41 % Picroside I and about 16.60% Picroside II.

### Example 3. Isolation of Picroside I and Picroside II of P. kurroa

The purified extract of *P. kurroa* prepared by the extraction method according to Example 2. Isolation was performed using normal silica column chromatography. 90 gm of silica (100-200#) was packed with chloroform into a glass column. 6 gm of purified extract was dissolved in sufficient amount of methanol. Further, 12 gm of silica (60-120#) was added for adsorption. This mixture was dried and loaded on silica bed. Multiple Fractions collection were started with 5% methanol in chloroform. Fractions were pooled together according to TLC pattern.

Component analysis was performed using HPLC. HPLC result confirmed that the Picroside I of purity of about 96.42% and Picroside II of purity of about 89.02% were obtained.

### Example 4. Preparation of Picroside I tablet

### Preparation of tablet

The purified Picroside I prepared by the methods according to Example 1, 2, and 3 was used for the said oral composition. Tablet was prepared by dissolving Picroside I in sufficient quantity of ethanol (5 mL for 50 tablets batch size). F-melt Type C was previously sifted through #40 sieve, and granulated using the previous step. Granulated wet mass, thus obtained, was dried at 60°C. Dried granules were then sifted using ASTM #40 and lubricated with ASTM #60. Magnesium Stearate was passed manually. Tablets were compressed using below mentioned parameters: Punch Dimension: 5.5 mm standard Concave; Target weight: 70 mg; Hardness: 30-50 N; D.T: 1-2 Min; Thickness: 2.6±0.2 mm; Friability: NMT 1%

**Table 1: Composition of Picroside I Tablet (1 mg)**

| **Ingredients** | **mg/tablet** | **w/w%** |
|---|---|---|
| Picroside I | 1.0 | 1.43 |
| F melt Type C | 68.50 | 97.86 |
| Ethanol | qs | |
| Magnesium stearate | 0.5 | 0.71 |

The above method including process parameters and ingredients ratio were replicated to prepare tablet formulation of Picroside II. 1 mg Picroside II was added to the composition instead of 1 mg Picroside I.

### Example 5. Preparation and stability test of Picroside I injectable formulation

### Preparation of injection

The purified Picroside I prepared by the methods according to Example 1, 2, and 3 was used for the said injection composition. Injection preparation was prepared by dissolving 1 mg Picroside I in solvent, controlling pH to about 4-7and then filling in ampules followed by subsequent sterilizing by conventional injection preparation method. For comparative compositions, different solvents such as methanol, 2.5% PEG400, 5% PEG400, 5% DMSO, 5% ethyl lactate, and 1.4% propylene glycol were used for Picroside I solubility.

The solutions were kept for sonication for 15 min and left the solutions stand at room temperature for 1 hour. HPLC was used for quantitative analysis. Composition prepared with 2.5% PEG400 were found to be the most suitable and were further analyzed for stability.

The above method including process parameters and ingredients ratio were replicated to prepare injectable formulation of Picroside II. 1 mg Picroside II was added to the composition instead of 1 mg Picroside I.

### Stability Data

Three compositions of 1 mg Picroside I and 2.5% PEG400 were kept for sonication for 15 min and left to stand at room temperature (RT), 2-8°C and 40°C, 75%RH for 1 month. and injected (20µL) into HPLC at 220nm for quantitative analysis. All compositions exhibited excellent stability over a period of 1 month.

**Table 2: Stability analysis of injectable composition of Picroside I and 2.5% PEG400; analyzed by HPLC quantitative analysis.**

| **Temperature conditions** | **% Result (1 month)** |
|---|---|
| 2-8°C | 96.59 |
| RT | 102.33 |
| 40°C, 75% RH | 86.89 |

### Example 6. Animal model test (mice)

In order to determine the anti-neutropenia effect of Picroside I test was performed by using neutropenia induced mice.

### Preparation of Experiment animal

In-house bred Swiss albino mice were housed under standard laboratory conditions, such as, air-conditioned with adequate fresh air supply (air changes 12-15 per hour), room temperature of 21.1-23.2°C and relative humidity of 55-69 %, with 12 hours light and 12 hours dark cycle were strictly maintained in the experimental room. The mice were acclimatized for a minimum period of seven days to laboratory conditions.

### Cyclophosphamide administration to induce neutropenia

The selected Swiss albino mice were randomized into four groups based on body weight, each group comprising of 6 mice. Group 2 and 3, 4 animals were treated with 150 mg/kg of cyclophosphamide through intraperitoneal route on Day 0 to initiate inducing neutropenia. On Day 3, the animals of group 2, 3 and 4, reflected the cyclophosphamide induced neutropenication when compared with Group 1 (Control) mice. On day 4, mice from Groups 2, 3 and 4 were again injected with a single dose of 100 mg/kg of cyclophosphamide. Total and differential count (neutrophils, lymphocytes, and monocytes) was recorded on day 3. The neutrophil count on day 3 was compared between groups using One-way ANOVA followed by Dunnett's test or student 't' test. P<0.05 was considered statistically significant.

**Table 3: mean %-WBC and %-Neutrophil count on Day 3 of administering cyclophosphamide**

| **Groups** | **% WBC** | **% Neutrophil** |
|---|---|---|
| Group 1 | 6.52 | 25.83 |
| Group 2 | 1.78 | 19.33 |
| Group 3 | 1.53 | 20.83 |
| Group 4 | 2.00 | 21.83 |

### Example 7. Administration of Picroside I (injectable) to mice with cyclophosphamide induced neutropenia

Cyclophosphamide related neutropenia was induced in mice subjects using method according to Example 6. Injectable composition was prepared using the method according to Example 5. The Picroside I composition was administered through subcutaneous route at a dose of 1 mg/mouse/day to the animals in Group 3 for 5 days respectively starting from day 5 ending at day 10. Picroside II composition was administered through subcutaneous route at a dose of 1 mg/mouse/day to the animals in Group 4 for 5 days respectively starting from day 5 ending at day 10. On day 10, again blood was collected to count the total leucocyte count and differential count (neutrophils, lymphocytes, and monocytes) manually. The neutrophil count on day 10 was compared between groups using One-way ANOVA followed by Dunnett's test or student 't' test. P<0.05 was considered statistically significant. Additionally, the mice were observed for changes in body weight and any clinical symptoms.

There were no treatment related changes in body weight in any of the subject mice treated with Picroside I or Picroside II. There were also no clinical signs of toxicity and mortality observed in any of the subject mice treated with Picroside I or Picroside II.

Day 10 results revealed that cyclophosphamide showed neutropenia and decreased total lymphocyte count in Group 2 (marked Vehicle control) as compared with Group 1 (marked Normal control). Treatment to subjects of Group 3 and Group 4 with Picroside I and Picroside II respectively showed significant increase in both Total leukocyte count and neutrophil count on Day 10 as compared to Group 2, indicating total reversal of neutropenia. Moreover, Picroside I and Picroside II increased neutrophil count significantly (P<0.01) more than that of Group 1 (Normal control) mice.

Based on the above results it is evident that injection of Picroside I and Picroside II significantly reverses cyclophosphamide induced neutropenia in mice.

**Table 4: mean %-Neutrophil and WBC count on Day 10 of Picroside I and Picroside II**

| **Groups** | **% Neutrophil (day 3)** | **% Neutrophil (day 10)** | **% WBC (day 3)** | **% WBC (day 10)** |
|---|---|---|---|---|
| Group 1 | 25.83 | 26.17 | 6.52 | 6.37 |
| Group 2 | 19.33 | 20.17 | 1.78 | 3.08 |
| Group 3 (Picroside I) | 20.83 | 32.67 | 1.53 | 9.23 |
| Group 4 (Picroside II) | 21.83 | 32.83 | 2.00 | 6.98 |

### Example 8. Administration of Picroside I (oral) to mice with cyclophosphamide induced neutropenia

Cyclophosphamide related neutropenia was induced in mice subjects using method according to Example 6. Oral tablet composition was prepared using the method according to Example 4. The Picroside I composition was administered orally at a dose of 2 mg/mouse/day (2 tablets) to the animals in Group 1 and Group 2 for 5 days respectively starting from day 5 ending at day 10. For a comparative study, oral composition was administered using distilled water and PEG. Specifically, oral tablets were administered in distilled water to mice in Group 1 and in 5% PEG to mice in Group 2. On day 10, again blood was collected to count the total leucocyte count and differential count (neutrophils, lymphocytes, and monocytes) manually. The neutrophil count on day 10 was compared between groups using One-way ANOVA followed by Dunnett's test or student 't' test. P<0.05 was considered statistically significant. Additionally, the mice were observed for changes in body weight and any clinical symptoms.

There were no treatment related changes in body weight in any of the subject mice treated with Picroside I. There were also no clinical signs of toxicity and mortality observed in any of the subject mice treated with Picroside I.

Day 10 results revealed that treatment to subjects of Group 1 with Picroside I in distilled water and of Group 2 with Picroside I in 5% PEG showed significant increase in both Total leukocyte count and neutrophil count on Day 10 as compared that on Day 3.

Based on the above results it is evident that Picroside I oral tablets significantly reverses cyclophosphamide induced neutropenia in mice.

**Table 5: mean Neutrophil and WBC count on Day 10 of Picroside I**

| **Groups** | **Neutrophil (10⁹/L) (day 3)** | **Neutrophil (10⁹/L) (day 10)** | **WBC (10⁹/L) (day 3)** | **WBC (10⁹/L) (day 10)** |
|---|---|---|---|---|
| Group 1 | 0.38 | 1.58 | 1.75 | 9.5 |
| Group 2 | 0.31 | 1.03 | 2.01 | 8.9 |

### Example 9. Comparative administration of Picroside I and G-CSF to mice with cyclophosphamide induced neutropenia

Cyclophosphamide related neutropenia was induced in mice subjects using method according to Example 6. Group 1 was marked vehicle control group, Group 2 was marked cyclophosphamide control, Group 3 was marked treated with Picroside I and Group 4 was marked treated with G-CSF treated. The Picroside I composition was administered through subcutaneous route at a dose of 1 mg/mouse/day to the animals in Group 3 for 5 days respectively starting from day 5 ending at day 10. The G-CSF composition was administered through subcutaneous route at a dose of 2 µg/mouse/day to the animals in Group 4 for 5 days respectively starting from day 5 ending at day 10. On day 10, again blood was collected to count the total leucocyte count and differential count (neutrophils, lymphocytes, and monocytes) manually. The neutrophil count on day 10 was compared between groups using One-way ANOVA followed by Dunnett's test or student 't' test. P<0.05 was considered statistically significant. Additionally, hepatotoxicity was observed by investigating ALT and AST levels.

Results showed treatment with Picroside I caused alleviation in cyclophosphamide induced decrease in % neutrophil. The observed changes in Picroside I treatment group were comparable to that of G-CSF treatment. Administration of Picroside I to the subject mice caused a significant reversal (p<0.001) of AST and ALT levels when compared to the Group 2. This observed decrease in AST and ALT was comparable to that of AST and ALT levels attained upon treatment with G-CSF.

**Table 6: mean %-Neutrophil count on Days 0, 3, and 10 after administration of Picroside I and G-CSF**

| **Groups** | **% Neutrophil (day 0)** | **% Neutrophil (day 3)** | **% Neutrophil (day 10)** |
|---|---|---|---|
| Group 1 (control) | 47.30 | 48.20 | 47.10 |
| Group 2 (CPH control) | 46.20 | 6.800 | 8.500 |
| Group 3 (Picroside I) | 46.00 | 6.600 | 23.90 |
| Group 4 (G-CSF) | 46.10 | 6.700 | 28.20 |

**Table 7: Effect of Picroside I in cyclophosphamide induced hepatotoxicity**

| **Groups** | **AST** (IU/L) | **ALT** (IU/L) |
|---|---|---|
| Group 1 (control) | 25.82 | 22.32 |
| Group 2 (CPH control) | 136.70 | 119.30 |
| Group 3 (Picroside I) | 50.32 | 37.16 |
| Group 4 (G-CSF) | 50.54 | 36.17 |

## Claims

1. A pharmaceutical composition comprising a therapeutically effective amount of purified Picroside I and acceptable pharmaceutical carriers or excipients, for use in the treatment of neutropenia.

2. The pharmaceutical composition of claim 1 comprising 100 to 400 mg of purified Picroside I and acceptable pharmaceutical carriers or excipients.

3. The pharmaceutical composition of claim 2, wherein the amount of purified Picroside I is 150 to 400 mg.

4. The pharmaceutical composition of claim 1, wherein the said composition is an oral or injectable composition.

5. The pharmaceutical composition of claim 4 wherein the said oral composition is in form of a tablet.

6. The pharmaceutical composition of claim 5 further comprising a filler, a solvent, a lubricant.

7. A pharmaceutical composition comprising 390 mg of purified Picroside I, a filler, a solvent, a lubricant, for use in the treatment of neutropenia; wherein said composition is ready for oral administration.

8. The pharmaceutical composition of claim 7, wherein the filler is F melt Type C, the solvent is ethanol, the lubricant is magnesium stearate.

9. The pharmaceutical composition of claim 7, wherein the amount of filler is about 98 percent by weight, the amount of lubricant is about 1 percent by weight.

10. The pharmaceutical composition of claim 4, wherein the said injectable composition is in form of an injection.

11. The pharmaceutical composition of claim 10 further comprising a solvent.

12. A pharmaceutical composition comprising 195 mg of purified Picroside I, and a solvent, for use in the treatment of neutropenia; wherein said composition is sterile and ready for subcutaneous administration having pH in the range of 4.0 to 7.0.

13. The pharmaceutical composition of claim 12, wherein the solvent is 2.5% PEG400.

14. The pharmaceutical composition of claim 12, wherein the composition is stored in a pre - filled sterile syringe or vial or cartridge.

15. The pharmaceutical composition of claim 1, wherein the composition is administered to the subject once a day.

16. The pharmaceutical composition of claim 1, wherein the composition is administered to the subject for up to 5 consecutive days.

## Patentansprüche

1. Pharmazeutische Zusammensetzung, umfassend eine therapeutisch wirksame Menge an gereinigtem Picrosid I und akzeptablen pharmazeutischen Trägern oder Hilfsstoffen, zur Verwendung bei der Behandlung von Neutropenie.

2. Pharmazeutische Zusammensetzung nach Anspruch 1, umfassend 100 bis 400 mg gereinigtes Picrosid I und akzeptable pharmazeutische Träger oder Hilfsstoffe.

3. Pharmazeutische Zusammensetzung nach Anspruch 2, wobei die Menge an gereinigtem Picrosid I 150 bis 400 mg beträgt.

4. Pharmazeutische Zusammensetzung nach Anspruch 1, wobei die Zusammensetzung eine orale oder injizierbare Zusammensetzung ist.

5. Pharmazeutische Zusammensetzung nach Anspruch 4, wobei die orale Zusammensetzung in Form einer Tablette vorliegt.

6. Pharmazeutische Zusammensetzung nach Anspruch 5, die ferner einen Füllstoff, ein Lösungsmittel und ein Gleitmittel umfasst.

7. Pharmazeutische Zusammensetzung, die 390 mg gereinigtes Picrosid I, ein Füllmittel, ein Lösungsmittel und ein Gleitmittel zur Verwendung bei der Behandlung von Neutropenie umfasst, wobei die Zusammensetzung zur oralen Verabreichung bereit ist.

8. Pharmazeutische Zusammensetzung nach Anspruch 7, wobei das Füllmittel F-Melt Type C, das Lösungsmittel Ethanol und das Gleitmittel Magnesiumstearat ist.

9. Pharmazeutische Zusammensetzung nach Anspruch 7, wobei die Menge an Füllstoff etwa 98 Gewichtsprozent beträgt und die Menge an Gleitmittel etwa 1 Gewichtsprozent beträgt.

10. Pharmazeutische Zusammensetzung nach Anspruch 4, wobei die injizierbare Zusammensetzung in Form einer Injektion vorliegt.

11. Pharmazeutische Zusammensetzung nach Anspruch 10, die ferner ein Lösungsmittel umfasst.

12. Pharmazeutische Zusammensetzung, umfassend 195 mg gereinigtes Picrosid I und ein Lösungsmittel zur Verwendung bei der Behandlung von Neutropenie, wobei die Zusammensetzung steril und zur subkutanen Verabreichung bereit ist und einen pH-Wert im Bereich von 4,0 bis 7,0 aufweist.

13. Pharmazeutische Zusammensetzung nach Anspruch 12, wobei das Lösungsmittel 2,5 % PEG400 ist.

14. Pharmazeutische Zusammensetzung nach Anspruch 12, wobei die Zusammensetzung in einer vorgefüllten sterilen Spritze oder einem vorgefüllten sterilen Fläschchen oder einer vorgefüllten sterilen Kartusche gelagert wird.

15. Pharmazeutische Zusammensetzung nach Anspruch 1, wobei die Zusammensetzung dem Patienten einmal täglich verabreicht wird.

16. Pharmazeutische Zusammensetzung nach Anspruch 1, wobei die Zusammensetzung dem Patienten bis zu 5 aufeinanderfolgende Tage lang verabreicht wird.

## Revendications

1. Une composition pharmaceutique comprenant une quantité thérapeutiquement efficace de Picroside I purifié et des supports ou excipients pharmaceutiques acceptables pour une utilisation dans le traitement de la neutropénie.

2. La composition pharmaceutique selon la revendication 1 comprenant 100 à 400 mg de Picroside I purifié et des supports ou excipients pharmaceutiques acceptables.

3. La composition pharmaceutique selon la revendication 2, dans laquelle la quantité de Picroside I purifié est de 150 à 400 mg.

4. La composition pharmaceutique selon la revendication 1, dans laquelle ladite composition est une composition orale ou injectable.

5. La composition pharmaceutique selon la revendication 4, dans laquelle ladite composition orale est sous la forme d'un comprimé.

6. La composition pharmaceutique selon la revendication 5 comprenant en outre un agent de charge, un solvant, un lubrifiant.

7. Une composition pharmaceutique comprenant 390 mg de Picroside I purifié, un agent de charge, un solvant, un lubrifiant, pour une utilisation dans le traitement de la neutropénie ; dans laquelle ladite composition est prête pour une administration par voie orale.

8. La composition pharmaceutique selon la revendication 7, dans laquelle l'agent de charge est du F melt type C, le solvant est de l'éthanol, le lubrifiant est du stéarate de magnésium.

9. La composition pharmaceutique selon la revendication 7, dans laquelle la quantité d'agent de charge est d'environ 98 pour cent en poids, la quantité de lubrifiant est d'environ 1 pour cent en poids.

10. La composition pharmaceutique selon la revendication 4, dans laquelle ladite composition injectable est sous la forme d'une injection.

11. La composition pharmaceutique selon la revendication 10 comprenant en outre un solvant.

12. Une composition pharmaceutique comprenant 195 mg de Picroside I purifié, et un solvant, pour une utilisation dans le traitement de la neutropénie ; dans laquelle ladite composition est stérile et prête pour une administration sous-cutanée, et a un pH compris entre 4,0 et 7,0.

13. La composition pharmaceutique selon la revendication 12, dans laquelle le solvant est du PEG400 à 2,5 %.

14. La composition pharmaceutique selon la revendication 12, dans laquelle la composition est stockée dans une seringue, un flacon ou une cartouche stérile prérempli(e).

15. La composition pharmaceutique selon la revendication 1, dans laquelle la composition est administrée au sujet une fois par jour.

16. La composition pharmaceutique selon la revendication 1, dans laquelle la composition est administrée au sujet pendant une durée maximale de 5 jours consécutifs.
